# EUROPÄISCHE PATENTSCHRIFT

(11) **EP 4 265 283 B1**
(45) Veröffentlichungstag und Bekanntmachung des Hinweises auf die Patenterteilung: **17.07.2024**
(21) Anmeldenummer: 23156815.5
(22) Anmeldetag: 15.02.2023
(51) Int. Cl.: A61M 1/34, A61M 1/00

(54) **VORRICHTUNG ZUM ENTSORGEN VON IN EINEM FILTRATBEUTEL GESAMMELTEM FILTRAT AUS EINER HÄMOFILTRATION**
DEVICE FOR REMOVING FILTRATE FROM HAEMOFILTRATION COLLECTED IN A FILTRATE BAG
DISPOSITIF POUR L'ÉLIMINATION DU FILTRAT D'HÉMOFILTRATION COLLECTÉ DANS UNE POCHE DE FILTRAT

(30) Priorität: 21.04.2022 AT 502662022
(43) Veröffentlichungstag der Anmeldung: 25.10.2023
(73) Patentinhaber: Hageneder, Konrad, 4643 Pettenbach (AT)
(72) Erfinder: Hageneder, Konrad, 4643 Pettenbach (AT)
(74) Vertreter: Hübscher & Partner Patentanwälte GmbH

(56) Entgegenhaltungen:
- WO-A1-2006/029435
- WO-A1-2021/089690
- US-A1- 2005 139 532

## Beschreibung

Die Erfindung bezieht sich auf eine Vorrichtung zum Entsorgen von in einem Filtratbeutel gesammeltem Filtrat aus einer Hämofiltration mit einem auf Stützrädern fahrbar abgestützten, selbsttragenden Behälter, der eine Austragseinrichtung für das eingefüllte Filtrat aufweist.

Zum Unterschied zu einer Hämodialyse wird bei einer Hämofiltration nicht ein Dialysat eingesetzt, in das die harnpflichtigen Substanzen diffundieren, sondern dem Blut über eine hochpermeable Membran Flüssigkeit entzogen, die als Filtrat in einem Filtratbeutel gesammelt und mit den darin enthaltenen harnpflichtigen Substanzen verworfen wird. Durch eine dem Blut nach der Hämofiltration zugeführte Substitutionslösung wird zumindest ein Teil der dem Blut entzogenen Flüssigkeit ersetzt.

Da im Vergleich mit der bei einer Dialyse zu entsorgenden Dialysatmenge das zu verwerfende Filtrat bei der Hämofiltration in einem erheblich größeren Volumenstrom anfällt, bedeutet dies, dass der an ein Gerät zur Hämofiltration angeschlossene Filtratbeutel in regelmäßigen Zeitabständen in mühsamer Handarbeit entsorgt werden muss.

Zum Entsorgen von Dialysaten ist es bekannt (WO 2006/029435 A1), einen selbsttragenden Behälter auf Stützrädern fahrbar abzustützen, der über eine verschließbare Füllöffnung mit dem Dialysat befüllt wird. Zur Austragung des Dialysats ist der Behälter mit einer entlang einer Behälterwand vom Behälterboden aufsteigenden, die Behälterwand durchsetzenden Austragsleitung versehen, die in einer außerhalb des Behälters in einer Anschlussmuffe drehbar gelagerten Ablaufleitung mündet. Über einen Druckluftanschluss im oberen Behälterbereich kann der Behälter mit einem Überdruck beaufschlagt werden, der das Dialysat durch die Austragsleitung aus dem Behälter drückt. Ein solcher Behälter ist selbstverständlich auch geeignet, das Filtrat aus den Filtratbeuteln eines Geräts zur Hämofiltration aufzunehmen, wodurch die Handhabung zur anschließenden Entsorgung des Filtrats erleichtert wird, nicht aber die mit dem Entleeren des Filtratbeutels in den Behälter erforderlichen Handhabungen.

Der Erfindung liegt somit die Aufgabe zugrunde, eine Vorrichtung zum Entsorgen von in einem Filtratbeutel gesammeltem Filtrat aus einer Hämofiltration so auszugestalten, dass insbesondere die Handhabung zum Entleeren der Filtratbeutel erleichtert wird.

Ausgehend von einer Vorrichtung der eingangs geschilderten Art löst die Erfindung die gestellte Aufgabe dadurch, dass der Behälter in seinem oberen Bereich ein mit einem Rückschlagventil versehenes, absperrbares Einlassventil mit einem Schlauchanschluss für einen an den Filtratbeutel anschließbaren Verbindungsschlauch sowie einen an eine Unterdruckquelle anschließbaren Druckanschluss umfasst.

Zufolge dieser Maßnahmen braucht der Filtratbeutel nicht mehr vom Gerät zur Hämofiltration abgenommen und entsorgt zu werden. Es genügt, zwischen dem Filtratbeutel und dem Einlassventil des Behälters einen Verbindungsschlauch zu legen, um den Filtratbeutel entleeren zu können, indem der Behälter mit Unterdruck beaufschlagt wird. Dieser Unterdruck sorgt für ein Überströmen des zu entsorgenden Filtrats aus dem Filtratbeutel durch den Verbindungsschlauch in den Behälter, ohne den Filtratbeutel vom Gerät abnehmen und entsorgen zu müssen, sodass der Filtratbeutel am Gerät verbleiben und nach jeder Entleerung erneut befüllt werden kann. Da in Anstalten, in denen Hämofiltrationen durchgeführt werden, üblicherweise Druckluftanschlüsse und Anschlüsse zu Unterdruckquellen für Krankenbetten zur Verfügung stehen, bedeutet die Unterdruckbeaufschlagung des Behälters zur Leerung des Filtratbeutels kaum einen Mehraufwand. Der Behälter ist zu diesem Zweck lediglich mit einem entsprechenden Druckanschluss zu versehen, um diesen Druckanschluss durch einen Beaufschlagungsschlauch mit einem vorgegebenen Anschluss zu einer Unterdruckquelle verbinden zu können.

Zur Entleerung des Behälters weist seine Austragseinrichtung eine vom Bodenbereich des Behälters ausgehende, entlang einer Behälterwand aufsteigende Austragsleitung mit einer außerhalb des Behälters zwischen einer an der Behälterwand anliegenden Ruhestellung und einer von der Behälterwand weggeneigten Auslaufstellung verschwenkbaren Ablaufleitung auf, sodass mit einer Druckbeaufschlagung des Behälters das Filtrat durch die Austragsleitung aus dem Behälter abfließen kann. Da für die Entleerung eine Druckbeaufschlagung und für die Befüllung eine Unterdruckbeaufschlagung des Behälters erforderlich ist, kann der für die Unterdruckbeaufschlagung vorgesehene Druckanschluss vorteilhaft auch für die Druckbeaufschlagung herangezogen werden. Zu diesem Zweck ist ja lediglich der Druckanschluss des Behälters wahlweise mit einer Unterdruckquelle oder einer Überdruckquelle zu verbinden. Mit einem gemeinsamen Druckanschluss sowohl für die Unterals auch die Überdruckbeaufschlagung erübrigt sich ein von der jeweiligen Beaufschlagungsart abhängiges Verschließen eines der sonst für die Unter- und Überdruckbeaufschlagung gesondert vorzusehenden Druckanschlüsse.

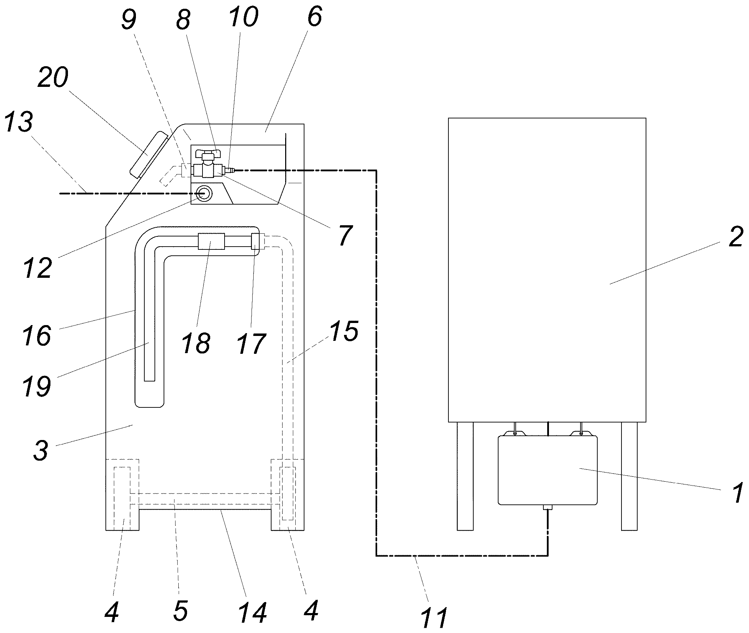

In der Zeichnung ist der Erfindungsgegenstand beispielsweise dargestellt, und zwar wird eine erfindungsgemäße Vorrichtung in einer schematischen Darstellung des Behälters in einer Vorderansicht gezeigt.

Die dargestellte Vorrichtung zum Entsorgen von in einem Filtratbeutel 1 gesammeltem Filtrat aus einer in einem entsprechenden Gerät 2 durchgeführten Hämofiltration weist einen selbsttragenden Behälter 3 in Form eines Kanisters auf, der auf Stützrädern 4 verfahren werden kann, die auf einer gemeinsamen, den Behälter 3 durchsetzenden Achse 5 gelagert sind. Der Behälter 3, der mit einem Tragegriff 6 versehen ist, ist mit einem Einlassventil 7 ausgerüstet, das mithilfe eines Betätigungshebels 8 geöffnet und gesperrt werden kann. Außerdem ist dem Einlassventil 7 ein Rückschlagventil 9 zugeordnet. An einen Schlauchanschluss 10 des Einlassventils 7 kann ein vom Filtratbeutel 1 ausgehender Verbindungsschlauch 11 angeschlossen werden.

Im oberen Bereich ist der Behälter 3 mit einem Druckanschluss 12 versehen, der durch einen Beaufschlagungsschlauch 13 an eine Unterdruckquelle angeschlossen werden kann. Die Beaufschlagung des Behälters 3 mit Unterdruck bewirkt bei geöffnetem, an den Filtratbeutel 1 angeschlossenem Einlassventil 7, dass Filtrat aus dem Filtratbeutel in den Behälter 3 abgesaugt und dadurch der Filtratbeutel entleert wird, ohne den auf der Unterseite des Gerätes 2 für die Hämofiltration befestigten Filtratbeutel 1 abnehmen zu müssen. Dies bedeutet auch, dass der Filtratbeutel 1 wiederholt gefüllt werden kann, ohne nach jeder Entleerung verworfen zu werden, wie dies erforderlich ist, wenn der Filtratbeutel 1 durch ein Abnehmen vom Gerät 2 von Hand entleert werden muss.

Der Behälter 3, der mehrere Füllungen des Filtratbeutels 1 aufnehmen kann, weist zu seiner Entleerung eine Austragseinrichtung auf, die eine vom Behälterboden 14 ausgehende, entlang einer Behälterwand aufsteigende Austragsleitung 15 umfasst. Diese Austragsleitung 15 durchsetzt die Behälterwand im Bereich einer Wandvertiefung 16 durch einen dichten Durchtritt 17. Im Bereich dieser Wandvertiefung 16 trägt die Austragsleitung 15 eine Lagermuffe 18 für eine absperrbare Ablaufleitung 19, die aus einer in der Wandvertiefung 16 aufgenommenen Ruhestellung in eine Auslaufstellung aus der Wandvertiefung 16 von der Behälterwand weggeschwenkt werden kann.

Die Austragung des im Behälter 3 gesammelten Filtrats erfolgt durch eine Druckbeaufschlagung des Behälters 3. Zu diesem Zweck wird der Druckanschluss 12 mit einer Überdruckquelle verbunden, vorzugsweise mithilfe eines Beaufschlagungsschlauchs 13, der auch für die Beaufschlagung des Behälters 3 mit Unterdruck eingesetzt werden kann. Da der Druckanschluss 12 sowohl für eine Unterdruck- als auch eine Überdruckbeaufschlagung des Behälters 3 herangezogen wird, kann dieser Druckanschluss 12 nicht mit einem Rückschlagventil versehen werden. Der Druckanschluss ist daher gesondert zu sperren, um den Behälter 1 dicht zu verschließen.

Um den Behälter nicht nur durch das Einlassventil 7 befüllen zu können, weist der Behälter 3 zusätzlich eine durch einen Deckel 20 verschließbare Füllöffnung auf.

## Patentansprüche

1. Vorrichtung zum Entsorgen von in einem Filtratbeutel (1) gesammeltem Filtrat aus einer Hämofiltration mit einem auf Stützrädern (4) fahrbar abgestützten, selbsttragenden Behälter (3), der eine Austragseinrichtung für das eingefüllte Filtrat aufweist, **dadurch gekennzeichnet, dass** der Behälter (3) in seinem oberen Bereich ein mit einem Rückschlagventil (9) versehenes, absperrbares Einlassventil (7) mit einem Schlauchanschluss (10) für einen an den Filtratbeutel (1) anschließbaren Verbindungsschlauch (11) sowie einen an eine Unterdruckquelle anschließbaren Druckanschluss (12) umfasst.

2. Vorrichtung nach Anspruch 1, **dadurch gekennzeichnet, dass** die Austragseinrichtung eine vom Bodenbereich des Behälters (3) ausgehende, entlang einer Behälterwand aufsteigende Austragsleitung (15) mit einer außerhalb des Behälters (3) zwischen einer an der Behälterwand anliegenden Ruhestellung und einer von der Behälterwand weggeneigten Auslaufstellung verschwenkbaren Ablaufleitung (19) umfasst und dass der Druckanschluss (12) des Behälters (3) wahlweise mit einer Unterdruckquelle oder einer Überdruckquelle verbindbar ist.

## Claims

1. Device for disposing a filtrate collected in a filtrate bag (1) from a haemofiltration with a self-supporting container (3) which is supported in a mobile manner on support wheels (4) and which has a discharge device for the poured in filtrate, **characterized in in that** the container (3) comprises in its upper region a shut-off inlet valve (7) and a pressure connection (12), wherein the shut-off inlet valve (7) is provided with a non-return valve (9) and has a hose connection (10) for a connecting hose (11) which can be connected to the filtrate bag (1), an wherein the pressure connection (12) can be connected to a vacuum source.

2. Device according to claim 1, **characterized in that** the discharge device comprises a discharge line (15) starting from the bottom region of the container (3) and rising along a container wall, with a run-off line (19) pivotable outside the container (3) between a rest position resting against the container wall and a run-off position inclined away from the container wall, and **in that** the pressure connection (12) of the container (3) can be connected either to a vacuum source or an overpressure source.

## Revendications

1. Dispositif pour l'élimination d'un filtrat d'hémofiltration collecté dans une poche de filtrat (1), comprenant un récipient autoporteur (3) supporté de façon mobile sur des roues de support (4), qui présente un dispositif d'évacuation pour le filtrat rempli, **caractérisé en ce que** le récipient (3) comprend, dans sa région supérieure, une vanne d'admission obturable (7) pourvue d'un clapet anti-retour (9), comprenant un raccord de tuyau (10) pour un tuyau de raccordement (11) pouvant être raccordé à la poche de filtrat (1), ainsi qu'un raccord de pression (12) pouvant être raccordé à une source de dépression.

2. Dispositif selon la revendication 1, **caractérisé en ce que** le dispositif d'évacuation comprend une conduite d'extraction (15) qui s'étend à partir de la région de fond du récipient (3) et qui monte le long d'une paroi du récipient, présentant une conduite d'évacuation (19) qui est capable de pivoter à l'extérieur du récipient (3) entre une position de repos appliquée contre la paroi du récipient et une position d'évacuation inclinée à l'écart de la paroi du récipient, et **en ce que** le raccord de pression (12) du récipient (3) peut être raccordé au choix à une source de dépression ou à une source de surpression.
